# FASCICULE DE BREVET EUROPEEN

(11) **EP 2 901 209 B1**
(45) Date de publication et mention de la délivrance du brevet: **14.08.2019**
(21) Numéro de dépôt: 13773195.6
(22) Date de dépôt: 25.09.2013
(51) Int. Cl.: G02C 13/00, A61B 5/103, A61B 3/11, A61B 3/14, A61B 5/00

(54) **PROCÉDÉ D'AIDE À LA DÉTERMINATION DE PARAMÈTRES DE VISION D'UN SUJET**
VERFAHREN ZUR BESTIMMUNG DER SICHTPARAMETER EINER PERSON
METHOD FOR HELPING DETERMINE THE VISION PARAMETERS OF A SUBJECT

(30) Priorité: 26.09.2012 FR 1259044
(43) Date de publication de la demande: 05.08.2015
(73) Titulaire: INTERACTIF VISUEL SYSTEME (I V S), 92110 Clichy (FR)
(72) Inventeur: THOMET, Pascal, F-75015 Paris (FR); ENCAOUA, David, F-78420 Carrieres Sur Seine (FR)
(74) Mandataire: Regimbeau
(86) Numéro de dépôt international: PCT/EP2013/069923
(87) Numéro de publication internationale: WO 2014/048961

(56) Documents cités:
- EP-A1- 1 747 750
- WO-A1-2010/119190
- DE-A1-102010 015 795
- DE-A1-102011 009 646
- FR-A1- 2 836 215
- FR-A1- 2 971 861
- US-A1- 2011 141 227

## Description

L'invention concerne l'analyse de la posture générale du visage du sujet et la prise de mesures effectuées par un opérateur, généralement un opticien, qui procède à l'acquisition de données nécessaires afin de déterminer la configuration générale d'implantation des verres correcteurs en regard des yeux du sujet, à des fins de personnalisation et d'optimisation des caractéristiques optiques de verres correcteurs ou de lentilles ophtalmiques qu'il doit porter, ainsi que leur montage sur une monture.

On connaît déjà de nombreux systèmes visant à optimiser la position des verres dans une monture par rapport à la position relative des pupilles des yeux du sujet et de la monture. A cet effet, des images fixes ou animées du visage portant la monture sont prises par une caméra, et la détection de la position des yeux est effectuée de même que la détection de l'emplacement de la monture.

En particulier, le document FR 2 860 887 au nom de la demanderesse divulgue un système dans lequel, à partir d'une série d'images animées du visage du sujet en cours de mouvement devant une caméra fixe, on détermine une image de référence dans laquelle le visage est le mieux axé sur la caméra, de manière à avoir la meilleure définition de la position relative des yeux et de la monture.

Parallèlement, des fabricants de verres ophtalmiques cherchent aujourd'hui à optimiser la conception de ces verres, notamment dans la technologie des verres dits progressifs, en examinant le comportement du sujet lorsque son regard se déplace. Par exemple, le document FR 2 892 529 au nom de la demanderesse divulgue un système comprenant :
- une caméra,
- un écran permettant de représenter les images prises par la caméra,
- un accessoire apte à être porté de façon fixe par le visage du sujet et portant une pluralité de repères visuels,
- des moyens formant cible(s) visuelle(s) susceptibles de couvrir au moins deux positions déterminées I1, I2 par rapport à la caméra, et
- des moyens d'analyse d'image capables d'analyser la position des repères visuels dans les images prises par la caméra. Les moyens d'analyse d'image en déduisent alors la position et l'orientation dans l'espace de l'accessoire, et donc du visage du sujet, lorsqu'il observe différentes régions des moyens formant cible(s) visuelle(s), pour en déduire notamment des informations sur l'importance relative du mouvement du visage lors du déplacement de la vision d'une cible à l'autre, ainsi que l'importance relative du mouvement des yeux.

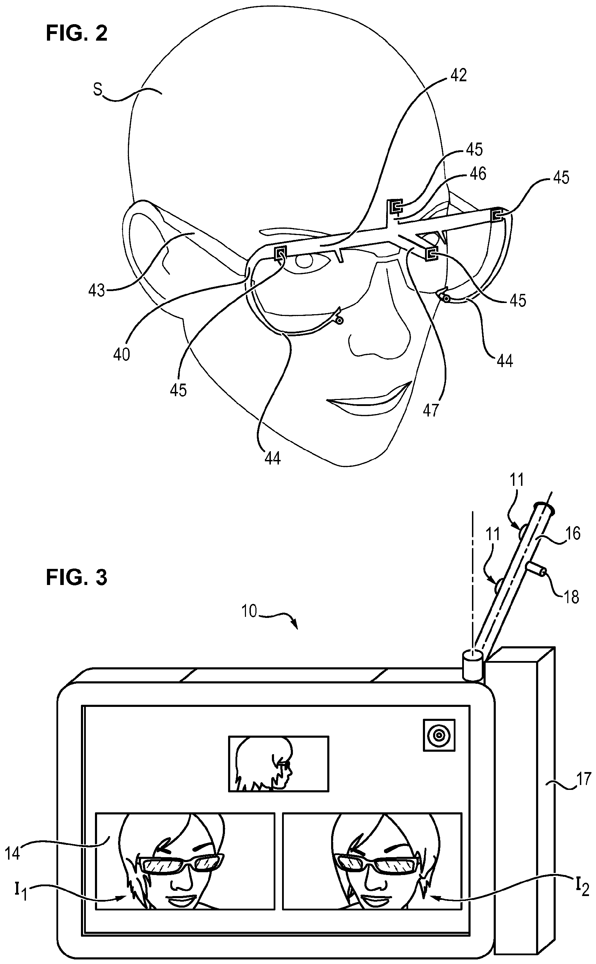

Typiquement, l'accessoire peut être conforme à l'accessoire illustré en figure 2 et comprendre des moyens formant indicateurs géométriques choisis spécifiquement pour mettre en valeur l'orientation de la monture. Il peut être constitué d'un support longiligne portant une série de repères visuels, placés le long des branches des montures et/ou sur le montant supérieur de face de la monture.

Le système permet alors de faire des mesures précises de paramètres tels que la position des pupilles, leur écart, etc. puisque le positionnement du sujet et des repères visuels ainsi que la direction de son regard (grâce aux cibles visuelles) sont connus et déterminés dans l'espace par rapport au système de mesure lui-même.

Pour que les mesures soient précises, il est en outre nécessaire que le visage du sujet soit dans une posture de référence, et regarde dans la position adaptée à la prise de mesures par le système.

La posture de référence qui est concernée par l'invention est la posture de vision de loin, dans laquelle le sujet se tient dans une position naturelle et fixe un point à l'infini droit devant lui suivant un plan horizontal. En variante, la posture de référence peut correspondre à une posture de vision de près, telle que la position de lecture, c'est-à-dire la position dans laquelle le sujet fixe un point à environ quarante centimètres de ses yeux et abaisse son regard de 30° par rapport au plan horizontal.

La posture du sujet peut par exemple être décrite (de manière non limitative) à l'aide de deux angles pour une direction du regard connue.

Le premier angle correspond au cap du visage, c'est à dire un angle orienté qui reflète le fait que le sujet a tendance à avoir le visage plus ou moins tourné à gauche ou à droite lorsqu'il regarde un objet placé droit devant lui.

Le deuxième angle correspond à l'inclinaison verticale du visage, c'est à dire un angle orienté qui reflète le fait que le sujet a tendance à avoir le visage plus ou moins relevé ou abaissé lorsqu'il regarde un objet placé droit devant lui. Pour une monture donnée, ce deuxième angle peut être la mesure de l'angle pantoscopique, c'est à dire la mesure de l'inclinaison du plan moyen de la lentille correctrice par rapport à la verticale.

Afin de déterminer la distance entre les pupilles du sujet (un des paramètres pour la fabrication des dispositifs de correction), l'opticien utilise généralement un pupillomètre. Dans ce cas, le cap est arbitrairement supposé nul puisque le pupillomètre est en appui contre le front du sujet. Néanmoins, ce dispositif ne permet pas de tenir compte de l'angle pantoscopique qui doit être mesuré séparément, ni, le cas échéant, du fait que le sujet puisse présenter un port de tête latéralisé (tendance du sujet à regarder plutôt à droite ou à gauche dans sa position de référence).

Cette approche ne garantit donc pas que la position prise par le sujet corresponde bien à sa posture naturelle. Cette posture naturelle est cependant d'une grande importance car elle détermine la manière dont le sujet va projeter son regard sur les verres de lunettes dans sa position de confort maximum. Une mauvaise posture conduit donc à de mauvaises mesures de centrage des verres. Il est donc primordial de choisir une image où la posture est correcte pour le calcul de la projection du regard sur les verres de lunette et donc d'évaluer la qualité de la position du sujet pendant la mesure.

De plus, l'ensemble des systèmes et procédés de mesure décrits ci-dessus ne permettent pas au sujet de regarder au loin droit devant à l'horizontale sans avoir de gêne visuelle, les dispositifs de vue étant placés dans le champ de vision du sujet lorsqu'il regarde en vision de loin. En effet, pour éviter des erreurs de perspectives, le dispositif de prise de vue doit être le plus proche possible de l'axe optique X du regard du sujet : il est donc nécessaire soit de placer le dispositif très loin du sujet (espace important pris dans le magasin et utilisation d'une caméra avec un zoom puissant), soit de simuler un point de visée plus éloigné du sujet que la caméra. Les cibles virtuelles sont donc souvent gênantes pour l'utilisateur. Par exemple, le reflet du sujet dans un miroir permet de doubler la distance entre le sujet et la cible visuelle, mais si le sujet est myope ou que le miroir n'est pas suffisamment immersif (par sa taille ou par la qualité de sa réflexion), il peut ensuite s'avérer difficile de cerner un point précis du visage du sujet. Le sujet peut donc difficilement adopter sa posture de vision de loin naturelle.

Ces systèmes sont en outre limités dans l'espace par la place disponible pour l'installation du système de mesure, de sorte que les cibles visuelles restent généralement proches du sujet, le forçant d'autant plus à contraindre son regard pour les fixer. Enfin, ces systèmes sont encombrants et nécessitent un espace minimum pour approximer la vision de loin.

Le document DE 10 2010 015795 décrit un dispositif de détermination des centrages d'un sujet, comprenant un écran, deux caméras disposées de part et d'autre de l'écran et un système de traitement. Les caméras sont de préférence non visibles par le sujet, tandis que des images choisies pour amener le sujet à prendre des positions particulières pour mesures des paramètres de vision sont diffusées sur l'écran. Plusieurs paramètres peuvent être déterminés, dont le coefficient tête-oeil, le centre de rotation de l'oeil en vision de près et de loin. Néanmoins, le procédé décrit dans ce dispositif impose au porteur de visualiser une cible déterminée, sur un écran placé en regard du sujet à une distance trop faible, ce qui contraint son regard et ne lui permet pas de se placer dans une posture de référence naturelle et regarder à l'infini. Par ailleurs, le système décrit dans ce document est très encombrant.

Le document EP 1 747 750 quant à lui décrit un procédé pour établir le comportement visuel d'un sujet, comprenant les étapes suivantes :
- munir le sujet d'un accessoire,
- faire asseoir le sujet, face à un écran. Le sujet doit être centré par rapport aux caméras, qui s'étendent de part et d'autre de l'écran, afin d'obtenir une carte stéréoscopique du visage du sujet,
- demander au sujet de suivre une image sur l'écran, et suivre le mouvement du visage, et
- déterminer le comportement du sujet à partir de ces images.

Ici encore, le procédé nécessite un espace important pour sa mise en oeuvre et contraint le sujet à fixer une cible disposée à une distance relativement proche, ce qui ne lui permet pas de prendre une posture de naturelle au cours des mesures.

Enfin, le document FR 2 971 861 décrit un procédé de détermination d'au moins un paramètre de vision d'un porteur, quelles que soient les conditions de lumière ambiante, au cours duquel on identifie la position d'un élément de calibrage du type damier sur une image de la tête du sujet et on utilise cette image pour ajuster la luminosité dans les images capturées suivantes. A cet effet, ce document propose un procédé au cours duquel on capture plusieurs images à l'aide d'une caméra. Le contraste est amélioré entre chaque capture, grâce aux éléments de calibrage détectés sur les images précédente. Ce procédé vise donc à améliorer la qualité des images capturées par le dispositif de prise de vues. Il nécessite cependant également un système encombrant, et les images du sujet sont prises de face ou de profil en contraignant a priori le regard du sujet lors de la mesure.

Un autre document pertinent de l'état de la technique est DE 10 2011 009646. Un objectif de l'invention est donc de proposer un nouveau procédé de mesure et un système associé, permettant de déterminer avec précision des paramètres de vision nécessaires à la fabrication d'un dispositif de correction de vue, qui ne contraigne pas le regard du sujet et soit plus polyvalent que les dispositifs connus à ce jour, et ce pour un coût modéré.

Par paramètres de vision, on comprendra ici notamment les paramètres compris dans la liste suivante : l'angle pantoscopique, la mesure des écarts/hauteurs pupillaires et la convergence des yeux dans cette mesure, la distance verre-oeil, le cap, le coefficient oeil-tête, etc.

Pour cela, l'invention propose un procédé d'aide à la détermination de paramètres de vision d'un sujet conforme à la revendication 1 annexée.

De la sorte, le dispositif de prise de vue ne constitue plus une gêne visuelle dans le champ de vision du sujet. Il ne coupe en effet plus l'axe du regard du sujet, de sorte qu'il lui est possible à présent de fixer une cible au loin à l'horizontal sans être contraint par l'espace ou le dispositif de mesure, et de limiter les erreurs liées au port de tête et/ou à la convergence des yeux dans la mesure des écarts/hauteurs pupillaires.

Certaines caractéristiques optionnelles mais non limitatives du procédé sont les suivantes :
* le dispositif de prise de vue est placé lors de la capture des images à l'extérieur d'une zone du champ de vision du sujet formant un cône ayant un angle au sommet de 5° à 30° par rapport à l'axe optique du sujet, de préférence entre 15° et 25°,
* l'une des images est prise face au sujet, vu de dessous,
* l'une des images est prise de côté.

Un tel placement du dispositif de prise de vues permet ainsi d'éviter de contraindre le regard et la position du sujet, en n'obstruant pas son champ de vision.
* au moins l'une des pupilles ou au moins l'un des reflets cornéens du sujet est visible sur chacune des images capturées,
* le procédé comprend en outre une étape au cours de laquelle on identifie des repères visuels dans les images, les repères visuels étant formés sur un accessoire apte à être porté de façon fixe par le visage du sujet,
* le dispositif de prise d'image est une caméra vidéo, et l'étape de capture des images comprend les sous-étapes suivantes :
   - filmer le visage du sujet de manière à obtenir une pluralité d'images du sujet selon des angles de vue différents, et
   - choisir parmi les images du sujet au moins deux images selon deux angles différents du visage du sujet,
* le procédé comprend en outre une étape au cours de laquelle on identifie des repères visuels dans les images, les repères visuels étant des points singuliers du visage du sujet,
* au moment de la capture des deux images, le visage du sujet est éclairé,
* au cours de l'étape de capture des images, on projette un faisceau lumineux sur un support en regard du sujet, par exemple un segment vertical,
* l'inclinaison et le cap du visage du sujet sont déterminés localement par le dispositif de prise de vue, sur un ordinateur local distinct du dispositif de prise de vue, ou à distance sur un serveur internet,
* le dispositif de prise de vues est une tablette numérique comprenant une caméra vidéo.

La possibilité de mettre en oeuvre un procédé d'aide conforme à l'invention permet donc d'adapter des tablettes numériques, qui peuvent être du commerce ou dédiées, pour la prise des mesures et la détermination des paramètres de vision du sujet.

D'autres caractéristiques, buts et avantages de la présente invention apparaîtront mieux à la lecture de la description détaillée qui va suivre, faite en référence aux figures annexées données à titre d'exemples non limitatif et sur lesquelles :

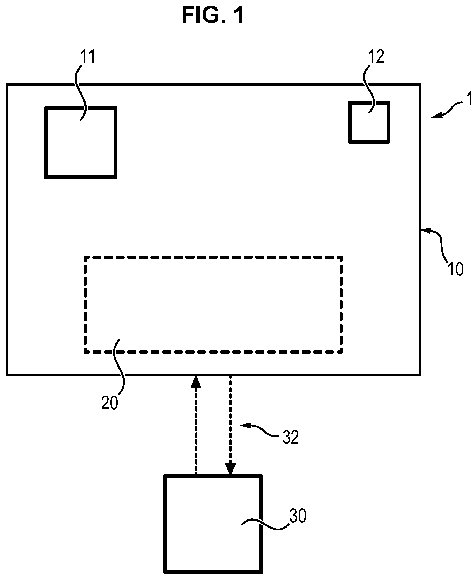

La figure 1 est un schéma d'un mode de réalisation d'un système aide à la détermination de paramètres de vision d'un sujet conforme à l'invention,
La figure 2 est un exemple d'accessoire pouvant être utilisé avec un système conforme à l'invention,
La figure 3 est un exemple de réalisation d'un dispositif de prise de vues pouvant être utilisé dans un système conforme à l'invention,
Les figures 4a et 4b illustrent un exemple de capture d'images conforme à l'invention,

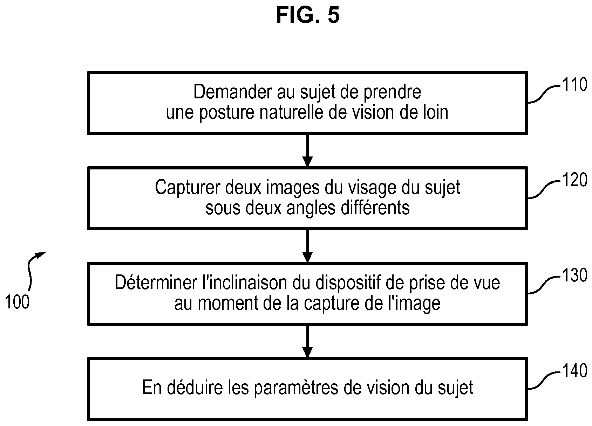

La figure 5 est un organigramme illustrant différentes étapes d'un exemple de réalisation d'un procédé aide à la détermination de paramètres de vision d'un sujet conforme à l'invention.

Un système 1 d'aide à la détermination de paramètres de vision d'un sujet S conforme à l'invention comprend notamment :
- un dispositif de prise de vues 10, adapté pour prendre des images du visage du sujet S, le visage du sujet comportant des repères visuels,
- un inclinomètre, solidaire en mouvement du dispositif de prise de vues, et
- des moyens de traitement, connectés au dispositif de prise de vues et à l'inclinomètre.

De manière générale, afin de déterminer les paramètres de vision du sujet, un opérateur (par exemple un opticien) prend au moins deux images I1, I2 du visage du sujet à des angles différents grâce au dispositif de prise de vues alors que le sujet regarde au loin, de manière à ne pas gêner le sujet lors de chacune des prises d'images I1, I2.

Pour cela, le dispositif de prise de vue est placé à distance de l'axe optique X du sujet, de préférence en dehors de sa zone fovéale, afin de ne pas obstruer son regard et garantir que le sujet S se place dans une posture naturelle et regarde au loin sans contrainte externe. Il est en effet important de ne pas obstruer la zone fovéale pour ne pas gêner le sujet S. Cependant, afin de réduire les risques de gêne, il peut être préférable de dégager une zone plus importante que la zone fovéale. Ainsi, selon une forme de réalisation, le dispositif de prise de vue 10 est placé, pour chacune des prises d'images I1 et I2, à l'extérieur d'une zone du champ de vision du sujet S formant un cône ayant un angle au sommet de 5° à 30° par rapport à l'axe optique X de chaque oeil du sujet S, de préférence entre 15° et 25° (le sommet du cône étant disposé sur l'axe optique au niveau du visage du sujet).

On comprendra en effet que plus le dispositif de prise de vue 10 est éloigné de l'axe optique X du sujet S, moins le sujet sera gêné par sa présence, la limite étant de pouvoir discerner correctement au moins l'une des pupilles (ou au moins un reflet cornéen) du sujet S.

Aucune cible visuelle n'est alors imposée au sujet S, de sorte qu'il peut adopter sa posture de vision de loin naturelle. Il est cependant possible à un opérateur de suggérer une telle cible au sujet S, afin par exemple d'inciter le sujet S à regarder au loin et droit devant lui. Une telle cible ne fait cependant pas partie du système 1, sa position n'étant pas définie par rapport au dispositif de prise de vue 10.

Les images et l'inclinaison du dispositif de prise de vues 10 sont alors analysées par les moyens de traitement qui en déduit des paramètres de vision du sujet.

### Le dispositif de prise de vues 10

Le dispositif de prise de vues 10 peut être une caméra vidéo, un appareil photographique, etc. Les images prises par le dispositif de prise de vues 10 peuvent notamment être des images numériques, afin de pouvoir être directement traitées par les moyens de traitement.

Le dispositif de prise de vues 10 possède de préférence un objectif grand angle, afin d'être capable de prendre une image complète du visage du sujet à une faible distance, c'est-à-dire entre dix centimètres et un mètre. La résolution du dispositif de prise de vues 10 dépend de la focale, de la distance à la personne, etc. et doit par ailleurs être suffisante pour pouvoir identifier des repères visuels 45 tels que des repères de couleur ou des points singuliers du visage. Par exemple, la résolution du dispositif de prise de vues 10 doit être suffisante pour que l'image des yeux comprenne au moins un pixel tous les 0.3 mm.

Le système 1 comprend en outre un inclinomètre 20, solidaire en mouvement du dispositif de prise de vues 10, adapté pour déterminer l'inclinaison du dispositif de prise de vues 10 par rapport à un plan horizontal. De préférence, l'inclinomètre 20 est intégré au dispositif de prise de vues 10. Il peut s'agir notamment d'un accéléromètre couplé à un circuit de traitement des signaux d'accélération. L'accéléromètre peut également être couplé à un gyroscope électronique afin d'améliorer la fiabilité de l'inclinomètre.

De manière optionnelle, afin notamment de mesurer le reflet cornéen du sujet, le système 1 peut en outre comprend un moyen d'éclairage 12 du visage du sujet, qui peut être solidaire du dispositif de prise de vues 10.

Par ailleurs, le dispositif de prise de vues 10 peut être mobile et portable par un opérateur afin de faciliter sa manipulation. Il peut en outre comprendre un écran 14, permettant à l'opérateur de visualiser les images prises par le dispositif de prise de vues 10.

Dans la forme de réalisation illustrée sur les figures, le dispositif de prise de vues 10 est par exemple une tablette numérique comprenant, au niveau de sa face arrière 16, une caméra vidéo 11 capable de prendre des images, ainsi qu'un accéléromètre 20. La tablette 10 peut en outre comprendre, au niveau de sa face avant, un écran 14 permettant de visualiser en temps réel les images prises par la caméra 11. Il peut s'agir par exemple d'une tablette commerciale du type iPad® ou Galaxy tab®, Iphone®, d'une tablette spécifiquement réalisée pour l'application à la détermination des paramètres de vision d'un sujet et comprenant un dispositif de prise de vues et un inclinomètre adapté, ou encore d'un téléphone intelligent.

Avantageusement, la caméra 11 peut alors être décalée par rapport au centre géométrique de la tablette 10, en étant par exemple disposée à proximité de l'un des bords de celle-ci, afin de faciliter la capture des images du sujet S tout en restant en dehors de sa zone fovéale, de préférence en dehors d'une zone du champ de vision formant un cône ayant un angle au sommet de 5° à 30° par rapport à l'axe optique X du sujet S, plus préférentiellement encore entre 15° et 25°, pour ne pas perturber sa posture naturelle de vision de loin.

De manière optionnelle, le flash de la tablette 10 peut être utilisé comme moyen d'éclairage 12 au moment de la prise de vues. La tablette 10 peut cependant être munie d'un dispositif d'éclairage 12 distinct du flash et rapporté sur celle-ci, notamment contre sa face arrière, afin d'éclairer le visage du sujet S au moment de la prise de vues.

Par ailleurs, le dispositif de prise de vues 10 peut en outre comprendre un projecteur 18 adapté pour projeter une cible visuelle contre un support en regard du sujet S. Par exemple, le projecteur 18 peut être un laser de faible puissance adapté pour projeter un faisceau lumineux sur une paroi.

Selon une autre forme de réalisation, le dispositif de prise de vues 10 peut être formé d'un écran 12 sur lequel est fixé, de manière amovible ou définitive, un bras 16 muni d'au moins une caméra 11. Le bras 16 peut être articulé sur l'un des bords de l'écran 14, par exemple le bord supérieur, autour d'un axe globalement perpendiculaire au bord de l'écran. Afin de ne pas gêner le sujet S lors des prises de vue, le bras 16 peut pivoter d'un angle supérieur à 90°, par exemple de l'ordre 120°. Le bras peut en outre pivoter (localement ou selon toute sa longueur) autour de son axe d'extension en entrainant avec lui la caméra 11, pour permettre de cadrer plus facilement le visage du sujet S. Par exemple, le bras 16 peut tourner sur lui-même selon un angle de l'ordre de 200°.

A titre d'exemple, le bras 16 peut mesurer une trentaine de centimètres et comprendre deux caméras 11 alignées le long de celui-ci et séparées d'environ dix centimètres. Cette forme de réalisation permet alors d'effectuer simultanément deux photographies I1, I2 du visage du sujet S selon deux angles de vue différents.

Dans cette forme de réalisation, le bras 16 muni de la(les) caméra(s) 11 peut alors être rapporté sur une tablette commerciale 10. L'inclinomètre 20 peut être fixé soit sur la tablette 10 (ou intégré à celle-ci), soit sur le bras 16 lui-même. Par ailleurs, la fixation du bras 16 peut être effectuée directement sur l'écran 10, ou via un adaptateur 17 (notamment dans le cas où l'écran est une tablette numérique du commerce).

### Les repères visuels

Les repères visuels peuvent être des points singuliers du visage, ou des repères visuels portés pas un accessoire 40 porté par le visage du sujet S.

L'accessoire 40 peut notamment être conforme aux accessoires décrits dans les documents FR 2 860 887 ou FR 2 892 529, illustré en figure 2. Cet accessoire 40, réalisé par exemple en matière plastique transparente, comprend un corps principal 42 allongé horizontalement avec deux branches latérales 43 également horizontales, s'étendant sensiblement à angle droit vers l'arrière par rapport au corps principal. Le corps principal 42 comprend le long de son bord inférieur deux petites pinces destinées à venir s'accrocher le long des bords supérieurs de deux verres de lunettes (ou sur une monture) portées par le sujet S, les branches 43 pouvant également comporter, de façon non illustrée, des aménagements pour se caler sur les branches de la monture. De la sorte, une fois que le sujet a mis des lunettes (correctrices ou optiquement neutres, ou encore la monture nue) dans une position bien stable, et que l'accessoire 40 a été mis en place, ledit accessoire occupe une position bien définie par rapport à le visage du sujet.

Selon un aspect de l'invention, l'accessoire peut en outre comprendre deux lames élastiques44, en métal ou en plastique, qui s'étendent depuis le bord inférieur du corps principal, et dont la forme incurvée est adaptée pour venir en appui contre le bord inférieur des verres de lunettes (ou de la monture). La forme des lames 44 est en outre adaptée pour que leur extrémité libre suive une trajectoire rectiligne et perpendiculaire au corps principal lorsque celles-ci sont déformées afin d'être fixées sur les verres (ou la monture). De la sorte, les lames 44 sont toujours fixées au même endroit sur les verres (ou la monture) au cours des mesures et ne perturbent pas le regard du sujet, quelle que soit la forme de sa monture.

Comme illustré sur la figure 2, l'accessoire 40 comporte un certain nombre de marqueurs ou repères visuels 45, ici huit zones carrées d'une couleur bien identifiée, par exemple un vert vif d'une longueur d'onde bien définie, deux repères visuels étant situés en des emplacements espacés bien définis sur l'une des branches 43, deux autres disposés symétriquement sur l'autre branche 43, et enfin quatre autres situés sur le corps principal. Plus précisément concernant ces derniers repères, le corps principal 42 de l'accessoire 40 comporte une partie en saillie vers le haut 46, dans la région supérieure de laquelle se trouvent un repère 45 et une partie en saillie vers l'avant 47, à l'extrémité libre de laquelle se trouve un repère 45. Les deux derniers repères 45 se trouvent à gauche et à droite, au voisinage du départ des branches 43.

En variante, les repères visuels 45 peuvent être des formes géométriques telles que des damiers, ou encore des repères émettant dans le spectre infrarouge.

Lorsque les repères visuels sont des points singuliers du visage, ces points singuliers peuvent notamment être les pupilles et/ou les reflets cornéens, ainsi que des points sur les bords de la monture du sujet.

### Moyens de traitement 30

Les moyens de traitement 30 sont adaptés pour analyser la position des repères visuels dans les images I1, I2 prises par le dispositif de prise de vues 10 ainsi que l'inclinaison du dispositif 10 déterminée par l'inclinomètre 20 au moment de chaque prise de vues, afin d'en déduire les paramètres de vision du sujet S.

Pour cela, les moyens de traitement 30 comprennent un processeur adapté pour recueillir et traiter les données en provenance du dispositif de prise de vues 10 (images I1, I2 du visage du sujet S) et de l'inclinomètre 20 (inclinaison du dispositif de prise de vues 10 par rapport au sol). Les résultats peuvent alors être affichés sur un écran du processeur ou le cas échéant sur l'écran 14 du dispositif de prise de vues 10.

Le processeur 30 peut être incorporé directement au dispositif de prise de vues 10. Cela peut notamment être le cas lorsque le dispositif de prise de vues 10 est une tablette numérique. L'ensemble des calculs est alors réalisé localement sur la tablette numérique10.

En variante, le processeur 30 peut être un ordinateur local distinct du dispositif de prise de vues 10, situé néanmoins à proximité de celui-ci. Le dispositif de prise de vues 10 transmet alors les données (images et angles) à l'ordinateur par un réseau 32 (sans fil ou filaire) qui réalise les calculs permettant d'obtenir les paramètres de vision du sujet S. Avantageusement, ce système 1 peut alors être incorporé en complément de systèmes de mesures existants, tels que ceux décrits dans les documents cités en introduction de la demande, et profiter ainsi des interfaces existantes (interfaces logiciel, de gestion, etc.) ce qui permet de réduire les coûts de développement, le prix d'achat du système lorsque les opticiens sont déjà munis de systèmes compatibles existants, et rassure l'opticien qui connaît déjà le matériel.

Dans cette variante de réalisation, lorsque le dispositif de prise de vues 10 est une tablette numérique, l'écran du processeur peut être affiché sur l'écran 14 de la tablette 10 par un système d'accès à un bureau distant, par exemple via un protocole VNC (acronyme anglosaxon de Virtual Network Computing) ou RDP (acronyme anglosaxon de Remote Desktop Protocole), permettant ainsi à l'opérateur de gérer l'ensemble des opérations directement depuis sa tablette 10.

Selon une autre variante encore, le processeur 30 peut être un serveur internet à distance, qui peut en outre être dédié. Les images I1, I2 et l'inclinaison du dispositif de mesure sont alors transmis (par un réseau sans fil ou filaire) au serveur à distance 30, qui renvoie alors les résultats soit directement au dispositif de prise de vues 10, notamment si celui-ci comprend un écran 14 pour les afficher, soit sur un ordinateur local connecté au serveur à distance.

Lorsque les repères visuels sont formés par des points singuliers du visage du sujet S, le processeur 30 comprend des moyens adaptés pour réaliser une reconstruction tridimensionnelle du visage du patient et effectuer à partir de cette reconstruction les mesures nécessaires pour déterminer les paramètres de vision du sujet.

Pour cela, on effectue une triangulation pour la reconstruction tridimensionnelle à partir de plusieurs images conformément à l'art antérieur. L'accessoire 40 permet alors de connaître au moins une distance connue entre deux points.

En variante, la reconstruction tridimensionnelle peut être réalisée par toute autre méthode de photogrammétrie ou stéréoscopie conventionnelle.

Dans la variante de réalisation mettant en oeuvre les points singuliers du visage, le système 1 comprend au moins deux dispositifs de prise de vues 10 dont la position et l'orientation dans l'espace l'un par rapport à l'autre sont connues. Il est alors possible de déterminer la position des points singuliers du visage et leur distance grâce aux images prises de préférence simultanément par les deux dispositifs de prise de vues 10 selon les techniques de photogrammétrie et de stéréoscopie décrites ci-dessus.

### Procédé

Nous allons à présent décrire un procédé d'aide à la détermination de paramètres de vision d'un sujet 100 conforme à l'invention. Le procédé 100 sera illustré dans ce qui suit avec un système 1 formé d'une tablette numérique 10 comprenant un écran, une caméra vidéo et un accéléromètre 20. Ceci n'est cependant pas limitatif, le procédé 100 pouvant être mis en oeuvre avec tous dispositifs de prise de vues 10 et inclinomètres 20 conformes à ce qui a été décrit plus haut.

Afin de réaliser les mesures, le sujet S se place tout d'abord dans sa posture naturelle de référence et regarde au loin 110. Pour cela, il se met à son aise et fixe un point à l'infini droit devant lui suivant un plan horizontal. Cette position n'est donc pas contrainte mais libre, dans la mesure où il n'a pas besoin de fixer une cible visuelle imposée ou de se regarder dans un miroir.

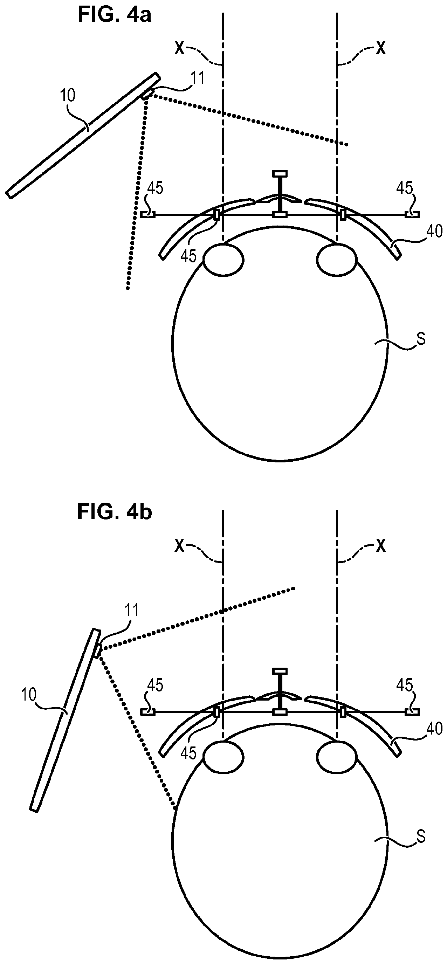

L'opérateur capture alors au moins deux images I1, I2 du visage du sujet à une faible distance 120, en cadrant le visage de sorte qu'au moins l'une des pupilles du sujet se trouve dans les deux images. De la sorte, il est alors possible d'effectuer les mesures au moins pour l'oeil correspondant. Etant donnés la faible distance entre la caméra 11 et le visage du sujet S (entre dix centimètres et un mètres), on obtient des images I1, I2 ayant une bonne résolution. Un exemple de placement du dispositif de prise de vues 10 et de la caméra vidéo 11 lors de la capture des images I1 et I2 par rapport au visage du sujet S et à son axe optique est illustré sur les figures 4a et 4b.

Lorsque le dispositif de prise de vue 10 est une caméra vidéo, il est possible soit de prendre les deux images I1 et I2 directement selon deux angles de prise de vue différents, soit de filmer le visage du sujet. Le film est réalisé de manière à prendre une pluralité d'images du visage du sujet S différentes et selon les angles de vue différents. On choisit ensuite parmi cette pluralité d'images deux images I1 et I2 prises selon deux angles de vue différents et convenant pour le procédé. Par exemple, il est possible de filmer le visage du sujet en déplaçant la caméra vidéo 10 entre une position latérale et une position du dessous du visage du sujet, en veillant à ne pas gêner son regard (c'est-à-dire en restant éloigné de sa zone fovéale). Ce choix d'images permet ainsi d'optimiser les images servant à déterminer les différents paramètres de vision du sujet S.

Dans le cas où les repères visuels 45 sont des points singuliers du visage, les images I1 et I2 sont prises de préférence simultanément par deux dispositifs de vues 10 distincts, dont la position et l'orientation l'un par rapport à l'autre sont connus.

De manière optionnelle, le moyen d'éclairage 12 peut être allumé pendant toute l'étape 120 de capture d'images, ou simplement au moment de chaque capture d'image. Dans le cas où les repères visuels sont des marqueurs 45 agencés sur un accessoire, le moyen d'éclairage 12 peut être disposé sur l'accessoire 40 lui-même plutôt que sur la tablette 10, afin d'éclairer les pupilles du sujet S.

Le cas échéant, l'opérateur peut s'aider de l'écran 14 de la tablette afin de bien cadrer le visage du sujet S.

Par exemple, l'opérateur prend une image I1 de face vue du dessous, de manière à ne pas pénétrer dans le champ de vision du sujet S et donc ne pas perturber sa posture de vision de loin naturelle, et une image I2 de côté, selon un angle inférieur à 90° pour que les deux pupilles soient visibles (par exemple de trois quarts).

En variante, l'opérateur peut prendre deux images I1, I2 de côté du visage selon des angles différents, par exemple à 20° et à 40° de l'axe de regard du sujet S, de manière à ne pas perturber son champ de vision. Ces images I1, I2 peuvent être obtenues successivement en déplaçant la tablette 10 entre les deux prises de vue I1, I2, ou simultanément lorsque la tablette 10 est équipée du bras 16 muni de deux caméras 11 espacées l'une de l'autre.

Au cours de ces mesures, les images I1, I2 sont alors enregistrées par la tablette numérique10, tandis que l'accéléromètre 20 détermine l'inclinaison de la tablette 10 au moment de chaque capture d'image 130.

Les images I1, I2 et l'inclinaison de la tablette 10 sont ensuite transmises au processeur 30, afin de déterminer la position respective des différents repères visuels dans les deux images I1, I2 et d'en déduire l'inclinaison naturelle et le cap naturel du visage du sujet 140, ainsi que des paramètres de vision tels que la convergence du sujet S.

La détermination des repères visuels peut être faite de manière automatique par le processeur 30 lui-même, ou manuellement par l'opérateur.

Une tablette numérique 10 étant généralement tactile, l'opérateur peut notamment visualiser et pointer les repères visuels directement sur les images I1, I2 affichées par l'écran 14. Il peut ainsi déterminer la position des iris, des pupilles, en appliquant les principes de photogrammétrie et de stéréoscopie. Ces informations sont ensuite utilisées par le processeur 30 pour en déduire la convergence du sujet S.

De manière optionnelle, lorsque le visage du sujet S est éclairé grâce aux moyens d'éclairage 12, le processeur 30 peut également déterminer la convergence du sujet S à partir de son reflet cornéen et de la position des iris selon les méthodes de calcul conventionnelles.

Ces résultats peuvent ensuite être affichés sur l'écran 14 de la tablette ou d'un ordinateur disposé à proximité.

Afin d'améliorer la mesure du cap naturel du sujet, il est possible de projeter une cible visuelle, de préférence un segment vertical, sur un support tel qu'un mur, au moyen d'un projecteur 18 fixé sur la tablette 10, et de demander au patient de regarder cette cible au moment des captures d'images. De la sorte, on force le sujet à placer sa tête dans la position correspondant à un cap nul.

La cible visuelle peut être un segment vertical afin de ne pas perturber la mesure de l'inclinaison du visage du sujet.

## Revendications

1. Procédé d'aide à la détermination de paramètres de vision d'un sujet (100), comprenant les étapes suivantes :
- capturer (120) deux images (I1, I2) selon deux angles différents du visage du sujet au moyen d'un seul dispositif de prise de vues (10), le sujet (S) étant dans une posture naturelle de vision de loin,
- déterminer (130) l'inclinaison par rapport au sol du dispositif de prise de vues (10) au moment de la capture des images au moyen d'un inclinomètre,
- en déduire (140) des deux images (I1, I2) et de l'inclinaison du dispositif de prise de vues (10) par rapport au sol l'inclinaison et le cap du visage du sujet (S),
**caractérisé en ce que** le dispositif de prise de vue est portable et mobile et est disposé en dehors d'une zone fovéale du sujet de sorte à ne pas couper son axe du regard au moment de la capture (120) des images (I1, I2).

2. Procédé selon la revendication 1, dans lequel le dispositif de prise de vue (10) est placé lors de la capture des images (120) à l'extérieur d'une zone du champ de vision du sujet (S) formant un cône ayant un angle au sommet de 5° à 30° par rapport à un axe optique (X) du sujet (S), de préférence entre 15° et 25°.

3. Procédé selon l'une des revendications 1 ou 2, dans lequel l'une des images (I1) est prise face au sujet, vu de dessous.

4. Procédé selon l'une des revendications 1 à 3, dans lequel l'une des images (I2) est prise de côté.

5. Procédé selon l'une des revendications 1 à 4, dans lequel au moins l'une des pupilles ou au moins l'un des reflets cornéens du sujet (S) est visible sur chacune des images (I1, I2) capturées.

6. Procédé selon l'une des revendications 1 à 5, comprenant en outre une étape au cours de laquelle on identifie des repères visuels (45) dans les images, les repères visuels (45) étant formés sur un accessoire (40) apte à être porté de façon fixe par le visage du sujet (S).

7. Procédé selon l'une des revendications 1 à 6, dans lequel le dispositif de prise d'image (20) est une caméra vidéo, et l'étape de capture des images (120) comprend les sous-étapes suivantes :
- filmer le visage du sujet de manière à obtenir une pluralité d'images du sujet selon des angles de vue différents, et
- choisir parmi les images du sujet au moins deux images (I1, I2) selon deux angles différents du visage du sujet (S).

8. Procédé selon l'une des revendications 1 à 5, comprenant en outre une étape au cours de laquelle on identifie des repères visuels (45) dans les images, les repères visuels (45) étant des points singuliers du visage du sujet (S).

9. Procédé selon l'une des revendications 1 à 8, dans lequel au moment de la capture des deux images (I1, I2), le visage du sujet (S) est éclairé.

10. Procédé selon l'une des revendications 1 à 9, dans lequel, au cours de l'étape de capture des images (I1, I2), on projette un faisceau lumineux sur un support en regard du sujet (S), par exemple un segment vertical.

11. Procédé selon l'une des revendications 1 à 10, dans lequel l'inclinaison et le cap du visage du sujet (S) sont déterminés localement par le dispositif de prise de vue, sur un ordinateur local distinct du dispositif de prise de vue, ou à distance sur un serveur internet.

12. Procédé selon l'une des revendications 1 à 11, dans lequel le dispositif de prise de vues (10) est une tablette numérique comprenant une caméra vidéo (11).

## Patentansprüche

1. Verfahren zur Unterstützung der Bestimmung von Sichtparametern einer Person (100), umfassend die folgenden Schritte:
- Erfassen (120) von zwei Bildern (I1, I2) gemäß zwei unterschiedlichen Winkeln des Gesichts der Person mittels einer einzigen Bildaufnahmevorrichtung (10), wobei die Person (S) in einer natürlichen Fernblickhaltung ist,
- Bestimmen (130) der Neigung der Bildaufnahmevorrichtung (10) in Bezug auf den Boden im Augenblick der Erfassung der Bilder mittels eines Neigungsmessers,
- Ableiten (140) daraus, aus den zwei Bildern (I1, I2) und der Neigung der Bildaufnahmevorrichtung (10) in Bezug auf den Boden, der Neigung und der Richtung des Gesichts der Person (S),
**dadurch gekennzeichnet, dass** die Bildaufnahmevorrichtung tragbar und beweglich ist und außerhalb einer fovealen Zone der Person derart angeordnet ist, dass ihre Blickachse im Augenblick der Aufnahme (120) der Bilder (I1, I2) nicht geschnitten wird.

2. Verfahren nach Anspruch 1, wobei die Bildaufnahmevorrichtung (10) bei der Aufnahme der Bilder (120) außerhalb einer Zone des Blickfelds der Person (S) platziert ist, das einen Konus mit einem Winkel an der Spitze von 5° bis 30°, vorzugsweise zwischen 15° und 25°, in Bezug auf eine optische Achse (X) der Person (S) bildet.

3. Verfahren nach einem der Ansprüche 1 oder 2, wobei eines der Bilder (I1) gegenüber der Person aufgenommen wird, gesehen von unten.

4. Verfahren nach einem der Ansprüche 1 bis 3, wobei eines der Bilder (I2) von der Seite aufgenommen wird.

5. Verfahren nach einem der Ansprüche 1 bis 4, wobei mindestens eine der Pupillen oder mindestens einer der Hornhautreflexe der Person (S) auf jedem der erfassten Bilder (I1, I2) zu sehen ist.

6. Verfahren nach einem der Ansprüche 1 bis 5, umfassend ferner einen Schritt, bei dem visuelle Bezugspunkte (45) in den Bildern identifiziert werden, wobei die visuellen Bezugspunkte (45) auf einem Zubehör (40) gebildet sind, das imstande ist, vom Gesicht der Person (S) fest getragen zu werden.

7. Verfahren nach einem der Ansprüche 1 bis 6, wobei die Bildaufnahmevorrichtung (20) eine Videokamera ist und der Schritt der Erfassung der Bilder (120) die folgenden Unterschritte umfasst:
- Filmen des Gesichts der Person derart, dass ein Vielzahl von Bildern der Person gemäß unterschiedlicher Blickwinkel erhalten wird, und
- Auswählen, aus den Bildern der Person, von mindestens zwei Bildern (I1, I2) gemäß zwei unterschiedlichen Winkeln des Gesichts der Person (S).

8. Verfahren nach einem der Ansprüche 1 bis 5, umfassend ferner einen Schritt, bei dem visuelle Bezugspunkte (45) in den Bildern identifiziert werden, wobei die visuellen Bezugspunkte (45) einzelne Punkte des Gesichts der Person (S) sind.

9. Verfahren nach einem der Ansprüche 1 bis 8, wobei im Augenblick der Erfassung der zwei Bilder (I1, I2) das Gesicht der Person (S) beleuchtet ist.

10. Verfahren nach einem der Ansprüche 1 bis 9, wobei während des Schritts der Erfassung der Bilder (I1, I2) ein Lichtstrahl auf einen Träger gegenüber der Person (S), beispielsweise ein vertikales Segment, projiziert wird.

11. Verfahren nach einem der Ansprüche 1 bis 10, wobei die Neigung und die Richtung des Gesichts der Person (S) lokal von der Bildaufnahmevorrichtung auf einem lokalen Rechner, der sich von der Bildaufnahmevorrichtung unterscheidet, oder beabstandet auf einem Internetserver bestimmt werden.

12. Verfahren nach einem der Ansprüche 1 bis 11, wobei die Bildaufnahmevorrichtung (10) ein digitales Tablet ist, das eine Videokamera (11) umfasst.

## Claims

1. A method for helping determine the vision parameters of a subject (100), including the following steps:
- capture (120) two images (I1, I2) at two different angles of the subject's face by means of an image capturing device (10), the subject (S) being in a natural distant vision posture,
- determine (130) the tilt with respect to the ground of the image capturing device (10) at the time of image capture with an inclinometer,
- deduce therefrom (140) the tilt and the cap angle value of the face of the subject (S), **characterized in that** the image capturing device is positioned outside a foveal area of the subject at the time of capture (120) of the images (I1, I2).

2. The method according to claim 1, wherein the image capturing device (10) is placed during image capture (120) outside an area of the field of vision of the subject (S), said area forming a cone having an apex angle of 5° to 30° with respect to an optical axis (X) of the subject (S), preferably between 15° and 25°.

3. The method according to one of claims 1 or 2, wherein one of the images (12) is taken in front of the subject, from below.

4. The method according to one of claims 1 to 3, wherein one of the images (12) is taken from the side.

5. The method according to one of claims 1 to 4, wherein at least one of the pupils or at least one of the corneal reflections of the subject (S) is visible on each of the captured images (I1, I2).

6. The method according to one of claims 1 to 5, further including a step during which visual markers (45) are identified in the images, the visual markers (45) being formed on an accessory (40) which is adapted to be fixedly worn on the face of the subject (S).

7. The method according to one of claims 1 to 6, wherein the image capturing device (20) is a video camera, and the step of capturing images includes the following sub-steps:
- filming the subject's face so as to obtain a plurality of images at different viewing angles, and
- choosing among the images of the subject at least two images (I1, I2) at two different angles of the subject's (S) face.

8. The method according to one of claims 1 to 5, further including a step during which visual markers (45) are identified in the images, the visual markers (45) being singular points of the subject's (S) face.

9. The method according to one of claims 1 to 8, wherein at the moment of capture of the two images (I1, I2), the face of the subject (S) is lit.

10. The method according to one of claims 1 to 9, wherein, during the step consisting of capturing the images (I1, I2), a beam of light is projected onto a support which faces the subject (S), for example a vertical segment.

11. The method according to one of claims 1 to 10, wherein the tilt and the cap angle value of the subject's face (S) are determined locally by the image capturing device, on a local computer distinct from the image capturing device, or remotely on an Internet server.

12. The method according to one of claims 1 to 11, wherein the image capturing device (10) is a digital tablet including a video camera (11).
